# DEMANDE DE BREVET EUROPEEN

(11) **EP 4 268 720 A1**
(43) Date de publication de la demande: **01.11.2023**
(21) Numéro de dépôt: 22170217.8
(22) Date de dépôt: 27.04.2022
(51) Int. Cl.: A61B 5/15

(54) **DISPOSITIF DE PRELEVEMENT DU SANG D'UN INDIVIDU, DISPOSITIF ET PROCEDE DE COLLECTE DU PLASMA CONTENU DANS LE SANG D'UN INDIVIDU**

(71) Demandeur: Bioulac, Bruno, 72250 Freudenstadt (DE)
(72) Inventeur: BIOULAC, Bruno, 72250 FREUDENSTADT (DE)
(74) Mandataire: Cabinet Bleger-Rhein-Poupon

(57) **Abrégé**

Dispositif de prélèvement (1) du sang (S) d'un individu, ce dispositif de prélèvement (1) comporte au moins une seringue (2) comportant un tube (3), un embout (4) qui équipe le tube (3), et un piston (5) qui comporte, d'une part, un joint d'étanchéité (6) qui est mobile à l'intérieur du tube (3) et, d'autre part, un moyen d'entraînement (7), qui est mobile par rapport au tube (3), qui est solidaire en déplacement dudit joint d'étanchéité (6), et qui est configuré pour entraîner ledit joint d'étanchéité (6) en déplacement par rapport au tube (3), caractérisé par le fait que ledit moyen d'entraînement (7) comporte au moins une portion (9) qui est sécable.

## Description

L'invention concerne un dispositif de prélèvement du sang d'un individu. Cette invention concerne, également, un dispositif et un procédé de collecte du plasma contenu dans le sang d'un individu.

L'invention concerne le domaine de la fabrication du matériel à usage médical, en particulier le domaine de la fabrication du matériel pour collecter du plasma contenu dans le sang d'un individu.

L'on connait, d'ores et déjà, un tel type de matériel médical.

Ce matériel comporte un dispositif de prélèvement du sang d'un individu qui comporte, usuellement, un tube de prélèvement, une aiguille et un système de raccordement de l'aiguille au tube de prélèvement.

Ce matériel comporte, également, une centrifugeuse qui est configurée pour recevoir une pluralité de tubes de prélèvement et pour entraîner ces tubes de prélèvement en rotation, ceci en sorte de séparer le plasma du reste du sang prélevé par centrifugation.

Un inconvénient d'une telle centrifugation consiste en ce que, pour séparer le plasma du reste du sang de manière appropriée, il est usuel d'ajouter au sang prélevé un anticoagulant qui est habituellement un produit chimique. L'ajout d'un tel produit chimique n'est, évidemment, pas souhaitable d'autant qu'au moins une partie de ce produit chimique se retrouve, au final, dans le plasma ce qui est préjudiciable à la qualité de ce plasma.

Ce matériel comporte, encore, un contenant de collecte du plasma ainsi qu'un système de transfert qui est configuré pour transférer, vers un tel contenant de collecte, le plasma du sang contenu dans le tube de prélèvement et obtenu par centrifugation. Un tel système de transfert comporte, usuellement, au moins une valve qui est traversée par le plasma. La traversée de cette valve par le plasma présente l'inconvénient de nuire à l'intégrité des plaquettes contenues dans le plasma

La présente invention se veut de remédier aux dispositifs de l'état de la technique.

A cet effet, l'invention concerne un dispositif de prélèvement du sang d'un individu. Ce dispositif de prélèvement comporte au moins une seringue comportant un tube, un embout qui équipe le tube, et un piston qui comporte, d'une part, un joint d'étanchéité qui est mobile à l'intérieur du tube et, d'autre part, un moyen d'entraînement, qui est mobile par rapport au tube, qui est solidaire en déplacement dudit joint d'étanchéité, et qui est configuré pour entraîner ledit joint d'étanchéité en déplacement par rapport au tube. Ce dispositif de prélèvement est caractérisé par le fait que ledit moyen d'entraînement comporte au moins une portion qui est sécable.

Selon une autre caractéristique, ladite au moins une portion sécable est configurée pour être sécable sous l'effet d'un mouvement du moyen d'entraînement autre qu'un mouvement d'entraînement du joint d'étanchéité par ce moyen d'entraînement.

Encore une autre caractéristique concerne le fait que ladite au moins une portion sécable comporte au moins une zone de fragilité et/ou au moins une zone de rupture préférentielle et/ou au moins une zone d'amorce de rupture.

Selon une autre caractéristique, le piston comporte des moyens de solidarisation qui sont configurés pour solidariser le joint d'étanchéité et le moyen d'entraînement tandis que le moyen d'entraînement comporte une partie d'extrémité proximale qui comporte une partie des moyens de solidarisation ainsi que ladite au moins une portion sécable.

L'invention concerne, également, un dispositif de collecte du plasma contenu dans le sang d'un individu. Ce dispositif de collecte comporte, d'une part, au moins un dispositif de prélèvement du sang d'un individu, d'autre part, au moins un contenant de collecte du plasma contenu dans le sang dudit individu et, d'autre part encore, au moins un système de transfert qui est configuré pour transférer le plasma du sang contenu dans ledit au moins un dispositif de prélèvement vers ledit au moins au moins un contenant de collecte. Ce dispositif de collecte est caractérisé par le fait que ledit au moins un dispositif de prélèvement du sang d'un individu présente les caractéristiques décrites ci-dessus.

Selon une autre caractéristique de l'invention, de dispositif de collecte se présente sous la forme d'un kit.

L'invention concerne, encore, un procédé de collecte du plasma contenu dans le sang d'un individu. Ce procédé de collecte comporte, d'une part, une étape de prélèvement du sang d'un individu, d'autre part, une étape de centrifugation du sang prélevé et, d'autre part encore, une étape de collecte du plasma contenu dans le sang prélevé et centrifugé. Ce procédé de collecte est caractérisé par le fait que, d'une part, l'étape de prélèvement consiste à prélever du sang à l'aide d'au moins un dispositif de prélèvement qui présente les caractéristiques décrites ci-dessus et, d'autre part, après l'étape de prélèvement et avant l'étape de centrifugation, le procédé de collecte comporte une étape de division qui consiste à diviser ladite au moins une portion sécable du moyen d'entraînement du piston de la seringue dudit au moins un dispositif de prélèvement et à retirer au moins une partie de ce moyen d'entraînement hors dudit au moins un dispositif de prélèvement..

Ainsi, l'invention concerne un dispositif de prélèvement qui comporte un moyen d'entraînement qui comporte au moins une portion qui est sécable.

La présence d'une telle portion sécable permet, avantageusement, après avoir prélevé du sang par l'intermédiaire du dispositif de prélèvement, après avoir divisé une telle portion sécable du moyen d'entraînement de ce dispositif de prélèvement, et après avoir retiré une partie de ce moyen d'entraînement, de positionner ce dispositif de prélèvement directement à l'intérieur d'une machine de centrifugation (notamment une machine de centrifugation à godets de type connu), ceci en sorte que son embout soit orienté vers le haut. Après centrifugation, le plasma occupe la partie supérieure du dispositif de prélèvement (qui comporte également ledit embout) ce qui permet, avantageusement, en maintenant le dispositif de prélèvement en sorte que son embout soit orienté vers le haut et en raccordant ce dispositif de prélèvement (plus particulièrement l'embout de ce dispositif de prélèvement) à un contenant de collecte par l'intermédiaire d'un système de transfert, de transférer immédiatement et uniquement le plasma contenu dans le dispositif de prélèvement (plus particulièrement contenu dans la partie supérieure de ce dispositif de prélèvement) vers un tel contenant de collecte.

Un tel transfert est opéré, avantageusement et aisément, en exerçant une pression (notamment ascendante) sur une partie du moyen d'entraînement qui est restée à l'intérieur du tube de la seringue du dispositif de prélèvement, notamment manuellement (plus particulièrement à l'aide d'un doigt) et/ou à l'aide d'une autre partie du moyen d'entraînement qui a été retirée préalablement. De plus, le fait que le plasma soit contenu dans la partie supérieure du dispositif de prélèvement (qui comporte également l'embout) permet, en maintenant le dispositif de prélèvement en sorte que son embout soit orienté vers le haut, de transférer le plasma vers le contenant de collecte sans soumettre ce plasma à des turbulences qui seraient préjudiciables à l'intégrité des plaquettes contenues dans ce plasma. Un tel transfert permet, également, d'envisager, dans un premier temps, d'éliminer au moins une fraction du plasma pauvre en plaquettes et, dans un deuxième temps, de collecter uniquement au moins une fraction de plasma riche en plaquettes. Ceci permet, avantageusement, de collecter du plasma de haute qualité.

Finalement, un tel transfert permet, également et avantageusement, de collecter l'intégralité du fibrinogène contenu dans le plasma.

Le dispositif de collecte peut comporter un unique contenant de collecte de plasma ainsi qu'une pluralité de dispositifs de prélèvement de sang qui peuvent être raccordés successivement à cet unique contenant de collecte. Ceci permet de collecter du plasma correspondant à du sang prélevé par l'intermédiaire d'une pluralité de dispositifs de prélèvement ce qui permet, avantageusement, d'homogénéiser un tel plasma.

D'autres buts et avantages de la présente invention apparaîtront au cours de la description qui va suivre se rapportant à des modes de réalisation qui ne sont donnés qu'à titre d'exemples indicatifs et non limitatifs.

La compréhension de cette description sera facilitée en se référant aux dessins joints en annexe et dans lesquels :

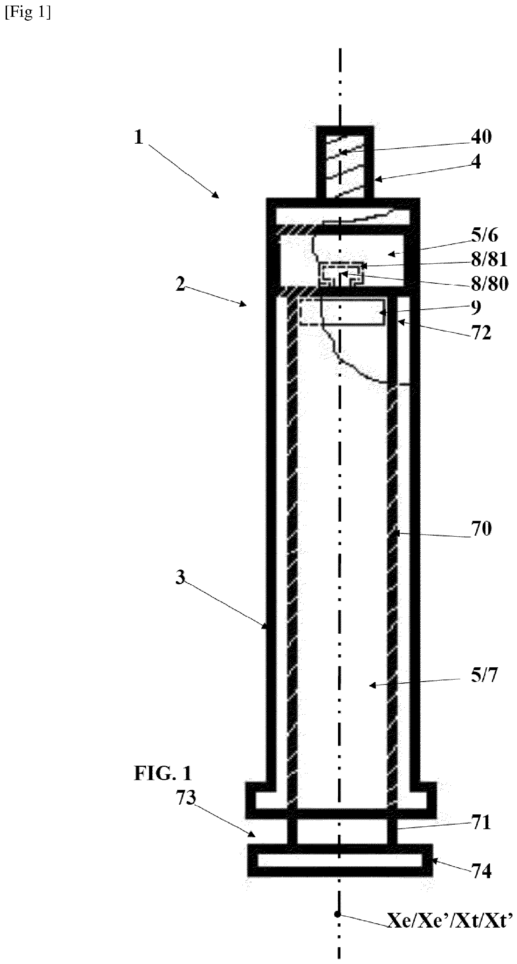

[Fig 1] est une vue schématisée, de face et en écorché d'un dispositif de prélèvement du sang d'un individu conforme à l'invention.

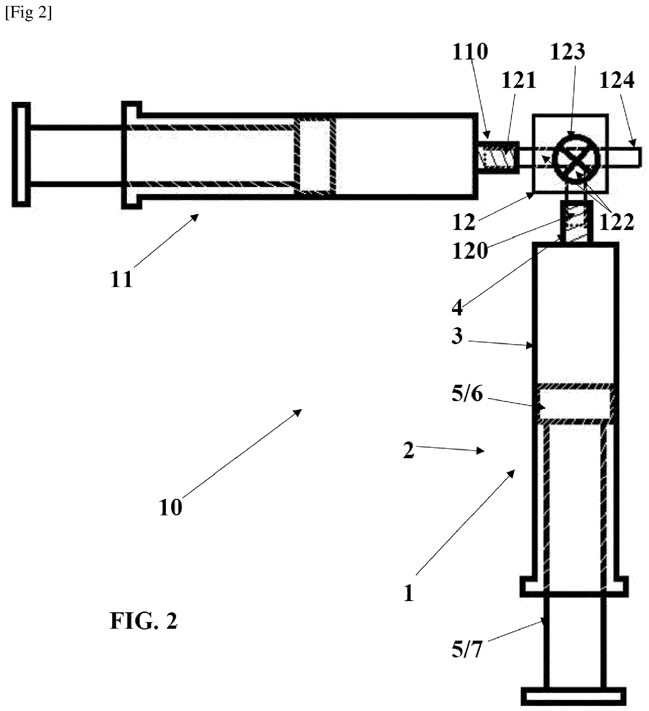

[Fig 2] est une vue schématisée et de face d'un dispositif de collecte du plasma contenu dans le sang d'un individu, ce dispositif de collecte étant conforme à l'invention.

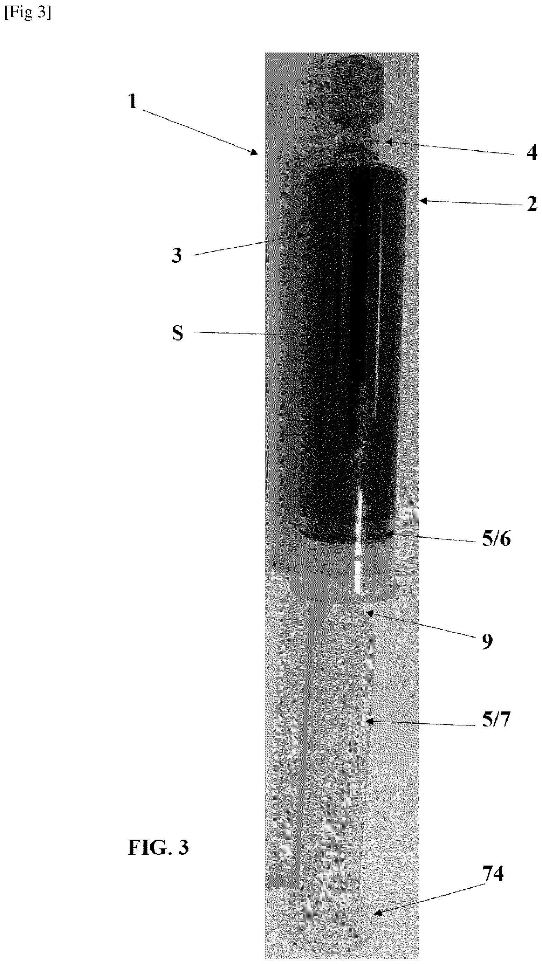

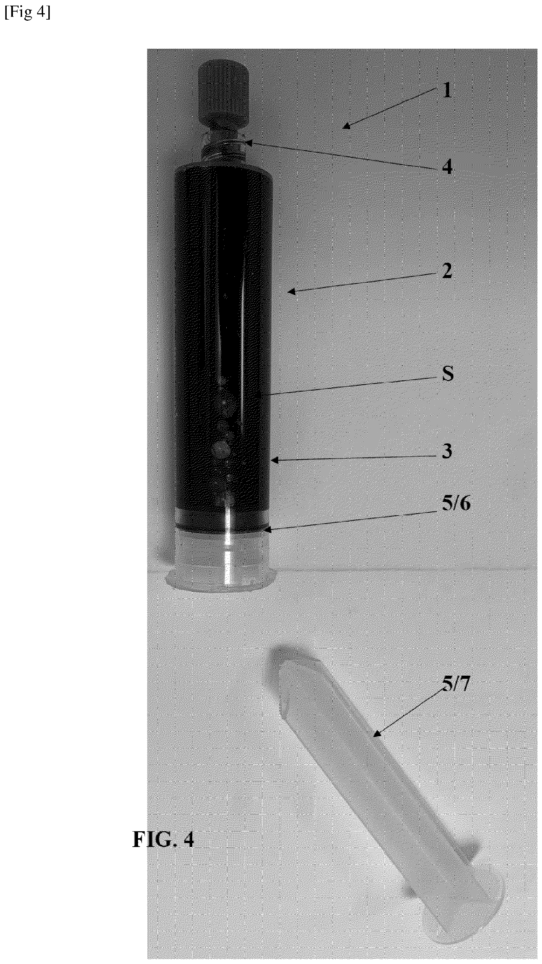

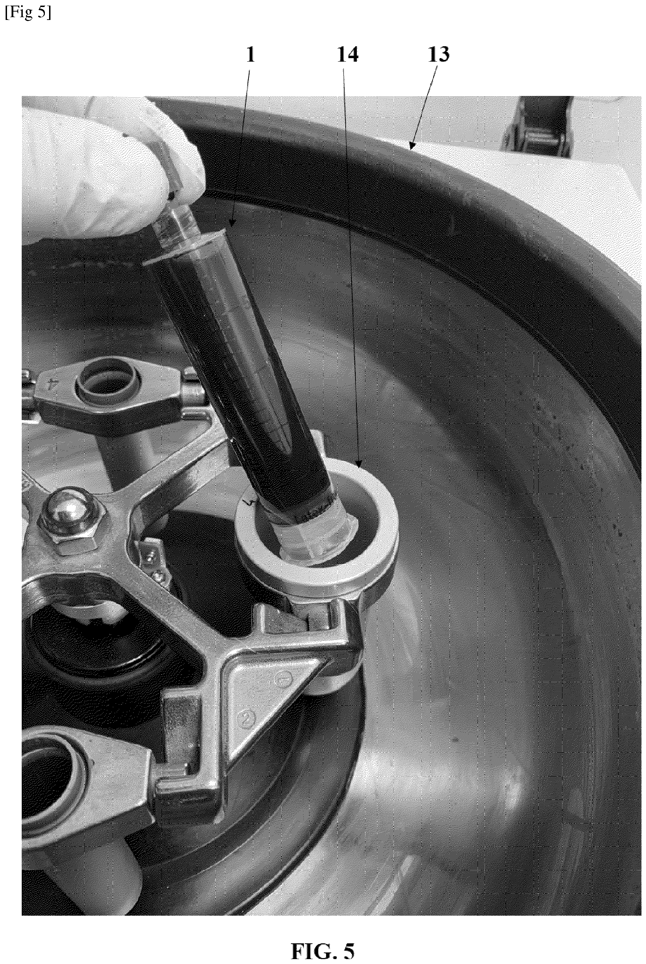

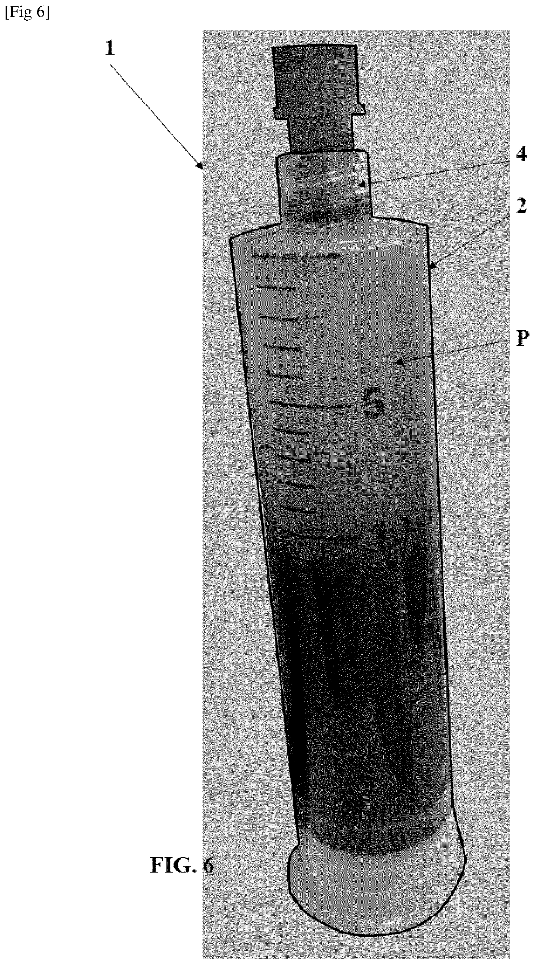

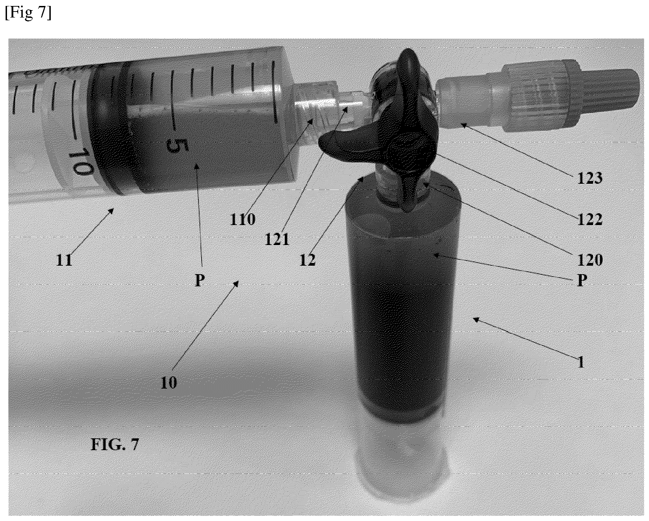

[Fig 3] à [Fig 7] sont des vues schématisées et de face, selon le cas, du dispositif de collecte conforme à l'invention, ceci au cours de différentes étapes du procédé de collecte du plasma contenu dans le sang d'un individu.

L'invention concerne le domaine de la fabrication du matériel à usage médical, en particulier le domaine de la fabrication du matériel pour prélever du sang chez un individu ainsi que pour collecter du plasma contenu dans le sang d'un tel individu.

La présente invention concerne, alors, un dispositif de prélèvement 1 du sang S d'un individu.

Ce dispositif de prélèvement 1 comporte au moins une seringue 2.

Une telle seringue 2 peut présenter une capacité de contenance, selon le cas, de 10 millilitres (mode de réalisation préféré) ou de 20 millilitres.

Une telle seringue 2 comporte, d'une part, un tube 3, qui s'étend selon un axe d'extension Xe, et qui est rigide et cylindrique.

D'autre part, cette seringue 2 comporte un embout 4 qui équipe le tube 3, plus particulièrement une extrémité de ce tube 3. Cet embout 4 est configuré pour être raccordé à un conduit flexible (notamment équipé d'un embout présentant un filetage externe apte à coopérer avec un filetage interne 40 que comporte ledit embout 4 de la seringue 2) qui équipe une aiguille ou qui est configuré pour équiper un cathéter.

D'autre part encore, cette seringue 2 comporte un piston 5 qui comporte un joint d'étanchéité 6 qui est mobile à l'intérieur dudit tube 3, plus particulièrement en translation selon un axe de translation Xt qui est au moins parallèle à (voire, et de préférence, confondu avec) l'axe de d'extension Xe du tube 3.

Ce piston 5 comporte, encore, un moyen d'entraînement 7 qui est configuré pour entraîner ledit joint d'étanchéité 6 en déplacement par rapport au tube 3.

Ce moyen d'entraînement 7 s'étend selon un axe d'extension Xe' qui est au moins parallèle à (voire, et de préférence, confondu avec) l'axe de d'extension Xe du tube 3.

Ce moyen d'entraînement 7 est mobile par rapport au tube 3, plus particulièrement en translation selon un axe de translation Xt' qui est au moins parallèle à (voire, et de préférence, confondu avec) l'axe d'extension Xe du tube 3 et/ou l'axe de d'extension Xe' de ce moyen d'entraînement 7 et/ou l'axe de translation Xt du joint d'étanchéité 6.

Ce moyen d'entraînement 7 est solidaire en déplacement dudit joint d'étanchéité 6,

A ce propos, on observera que le piston 5 comporte, alors, des moyens de solidarisation 8 qui sont configurés pour solidariser le joint d'étanchéité 6 et le moyen d'entraînement 7.

Selon une autre caractéristique, ce moyen d'entraînement 7 comporte au moins une partie interne 70 qui s'étend à l'intérieur du tube 3 ainsi qu'au moins une partie externe 71 qui s'étend à l'extérieur du tube 3.

Ce moyen d'entraînement 7 (plus particulièrement la partie interne 70 de ce moyen d'entraînement 7) comporte une partie proximale 72 qui comporte une partie 80 des moyens de solidarisation 8 qui coopère avec une autre partie 81 de ces moyens de solidarisation 8 que comporte le joint d'étanchéité 6.

Ce moyen d'entraînement 7 (plus particulièrement la partie externe 71 de ce moyen d'entraînement 7) comporte une partie distale 73 qui comporte un organe préhensible 74 qui est configuré pour être saisi (notamment manuellement), ceci en vue de déplacer ce moyen d'entraînement 7 (et, ainsi, le joint d'étanchéité 6) par rapport au tube 3, plus particulièrement en translation tel que décrit ci-dessus.

Selon l'invention, le moyen d'entraînement 7 comporte au moins une portion 9 qui est sécable.

Selon une caractéristique additionnelle, ladite au moins une portion sécable 9 est configurée pour être sécable sous l'effet d'un mouvement du moyen d'entraînement 7 autre qu'un mouvement d'entraînement du joint d'étanchéité 6 par ce moyen d'entraînement 7.

Tel que mentionné ci-dessus, le tube 3 et/ou le moyen d'entraînement 7 s'étend selon un axe d'extension (Xe ; Xe').

Selon une caractéristique alternative ou (et de préférence) additionnelle, ladite au moins une portion sécable 9 est, alors, configurée pour être sécable sous l'effet d'un effort (notamment de flexion) exercé sur ledit moyen d'entraînement 7, ceci selon une direction qui forme un angle non nul (notamment droit) avec un tel axe d'extension (Xe ; Xe').

Tel que mentionné ci-dessus, le joint d'étanchéité 6 et/ou le moyen d'entraînement 7 est mobile en translation selon un axe de translation (Xt ; Xt').

Selon une caractéristique alternative ou (et de préférence) additionnelle, ladite au moins une portion sécable 9 est, alors, configurée pour être sécable sous l'effet d'un effort (notamment de flexion) exercé sur ledit moyen d'entraînement 7, ceci selon une direction qui forme un angle non nul (notamment droit) avec un tel axe de translation (Xt ; Xt').

Selon une autre caractéristique, ladite au moins une portion sécable 9 est configurée pour être sécable manuellement.

Encore une autre caractéristique concerne le fait que ladite au moins une portion sécable 9 comporte au moins une zone de fragilité, notamment une zone d'épaisseur réduite.

Selon une caractéristique alternative ou (et de préférence) additionnelle, ladite au moins une portion sécable 9 comporte au moins une zone de rupture préférentielle, notamment comportant des perforations ou analogue.

Selon une caractéristique alternative ou (et de préférence) additionnelle, ladite au moins une portion sécable 9 comporte au moins une zone d'amorce de rupture, notamment comportant au moins une entaille, au moins une découpe ou analogue.

Encore une autre caractéristique concerne le fait que ladite au moins une portion sécable 9 est localisée à proximité (notamment à proximité immédiate) du joint d'étanchéité 6.

Tel que mentionné ci-dessus, le piston 5 comporte des moyens de solidarisation 8 tandis que le moyen d'entraînement 7 comporte la partie d'extrémité proximale 72 qui comporte la partie 80 de ces moyens de solidarisation 8 que comporte le moyen d'entraînement 7. C'est, plus particulièrement, cette partie d'extrémité proximale 72 qui comporte, alors, également, ladite au moins une portion sécable 9.

Encore une autre caractéristique concerne le fait que le moyen d'entraînement 7 comporte (plus particulièrement entre l'organe préhensible 74 et les moyens de solidarisation 8, notamment la partie 80 des moyens de solidarisation 8 que comporte le moyen d'entraînement 7) une tige ou une lame dont au moins une partie comporte et/ou constitue ladite au moins une portion sécable 9.

L'invention concerne, également, un dispositif de collecte 10 du plasma P contenu dans le sang S d'un individu.

Ce dispositif de collecte 10 comporte, d'une part, au moins un dispositif de prélèvement 1 du sang S d'un individu, d'autre part, au moins un contenant de collecte 11 du plasma P contenu dans le sang S dudit individu et, d'autre part encore, au moins un système de transfert 12 qui est configuré pour transférer le plasma P du sang S contenu dans ledit au moins un dispositif de prélèvement 1 vers ledit au moins au moins un contenant de collecte 11.

Dans ce dispositif de collecte 10, ledit au moins un dispositif de prélèvement 1 du sang S d'un individu présente les caractéristiques décrites ci-dessus. En particulier, un tel dispositif de prélèvement 1 comporte une seringue 2, qui présente les caractéristiques décrites ci-dessus, et qui peut présenter une capacité de contenance, selon le cas, de 10 millilitres ou de 20 millilitres.

Dans ce dispositif de collecte 10, ledit au moins un contenant de collecte 11 du plasma P comporte, chacun, au moins une seringue. Une telle seringue peut présenter une capacité de contenance, selon le cas, de 10 millilitres ou de 20 millilitres.

Selon un mode de réalisation préféré, le dispositif de collecte 10 comporte, d'une part, une pluralité de dispositifs de prélèvement 1 présentant, chacun, les caractéristiques décrites ci-dessus (en particulier comportant, chacun, une seringue 2 qui présente une capacité de contenance de 20 millilitres) et, d'autre part, un contenant de collecte 11 comportant une seringue de capacité de contenance de 20 millilitres.

Dans ce dispositif de collecte 10, ledit au moins un système de transfert 12 est configuré pour être raccordé audit au moins un dispositif de prélèvement 1 ainsi qu'audit au moins un contenant de collecte 11.

Ledit au moins un système de transfert 12 comporte, alors, au moins, d'une part, un premier raccord 120 qui est configuré pour être raccordé audit au moins un dispositif de prélèvement 1 (plus particulièrement à l'embout 4 que comporte un tel dispositif de prélèvement 1), d'autre part, un deuxième raccord 121 qui est configuré pour être raccordé audit au moins un contenant de collecte 11 (plus particulièrement à un embout 110, que comporte un tel contenant de collecte 11, et qui peut comporter un filetage interne) et, d'autre part encore, au moins un conduit de raccordement 122 qui raccorde le premier raccord 120 et le deuxième raccord 121.

De manière additionnel, ce système de transfert 12 peut, encore, comporter au moins un organe de commande 123 (plus particulièrement sous la forme d'au moins un robinet ou d'au moins une vanne, notamment à trois voies) qui est configuré pour être actionné (notamment de manière manuelle) en vue d'autoriser ou d'empêcher le transfert de plasma P.

Un tel système de transfert 12 peut, également, comporter un troisième raccord 124 qui est configuré pour être raccordé à un contenant de collecte additionnel (non représenté).

Selon une autre caractéristique de l'invention, ledit dispositif de collecte 10 peut se présenter sous la forme d'un kit.

Un tel kit est fermé et stérile ce qui rend le dispositif de collecte 10 intégralement adapté à une utilisation chirurgicale, notamment orthopédique.

L'invention concerne, encore, une installation de collecte du plasma P contenu dans le sang S d'un individu.

Une telle installation de collecte du plasma P comporte, d'une part, au moins un dispositif de collecte 10 qui présente les caractéristiques décrites ci-dessus et, d'autre part, une machine de centrifugation 13 du sang S prélevé par l'intermédiaire dudit au moins un dispositif de prélèvement 1 que comporte ledit au moins un dispositif de collecte 10.

Une telle machine de centrifugation 13 comporte, de préférence, des godets 14 qui sont, de préférence, dimensionnés et/ou configurés pour recevoir un dispositif de prélèvement 1 tel décrit ci-dessus.

L'invention concerne, également, un procédé de collecte du plasma P contenu dans le sang S d'un individu.

Ce procédé est, au moins en partie, mis en œuvre par le dispositif de collecte 10 et/ou par l'installation de collecte qui présente les caractéristiques décrites ci-dessus.

Ce procédé de collecte comporte :
- une étape de prélèvement du sang S d'un individu ;
- une étape de centrifugation du sang S prélevé ;
- une étape de collecte du plasma P contenu dans le sang S prélevé et centrifugé.

Ce procédé est caractérisé par le fait que l'étape de prélèvement consiste à prélever du sang S à l'aide d'au moins un dispositif de prélèvement 1 qui présente les caractéristiques décrites ci-dessus.

Ce procédé est, également, caractérisé par le fait que, après l'étape de prélèvement et avant l'étape de centrifugation, le procédé de collecte comporte une étape de division qui consiste à diviser ladite au moins une portion sécable 9 du moyen d'entraînement 7 du piston 5 de la seringue 2 dudit au moins un dispositif de prélèvement 1 et à retirer au moins une partie de ce moyen d'entraînement 7 hors dudit au moins un dispositif de prélèvement 1, plus particulièrement hors du tube 3 de la seringue 2 de ce dispositif de prélèvement 1.

Selon une autre caractéristique, dans ce procédé de collecte, l'étape de centrifugation du sang S consiste à centrifuger le sang S (plus particulièrement par l'intermédiaire d'une machine à centrifuger 13 telle que décrite ci-dessus) avec une accélération comprise entre 200g et 700g.

A ce propos, on observera que, lorsque ledit au moins un dispositif de prélèvement 1 comporte, chacun, une seringue 2 qui présente une capacité de contenance de 10 millilitres, l'accélération est comprise entre 200g et 500g, de préférence comprise entre 300g et 400g, notamment de l'ordre de 360g. Cependant, lorsque ledit au moins un dispositif de prélèvement 1 comporte, chacun, une seringue 2 qui présente une capacité de contenance de 20 millilitres, l'accélération est comprise entre 400g et 700g, de préférence comprise entre 500g et 600g, notamment de l'ordre de 550g.

Une telle accélération permet d'éviter d'ajouter au sang S prélevé un anticoagulant (notamment sous la forme d'un produit chimique) et/ou d'éviter de traumatiser les plaquettes contenues dans le sang S (prélevé et centrifugé) et, donc, dans le plasma P collecté.

Une autre caractéristique concerne le fait que la centrifugation est assurée pendant une durée comprise entre 6 et 10 minutes, de préférence de l'ordre de 8 minutes.

Selon une autre caractéristique, au cours de cette étape de centrifugation, ledit au moins un dispositif de prélèvement 1 est positionné à l'intérieur de la machine de centrifugation 13 (plus particulièrement à l'intérieur d'un godet 14 de cette machine de centrifugation 13) ceci en sorte que l'embout 4 de la seringue 2 dudit au moins un dispositif de prélèvement 1 soit orienté vers le haut.

En ce qui concerne l'étape de collecte du plasma P, celle-ci consiste, d'une part, en une étape de raccordement d'au moins un dispositif de prélèvement 1 (plus particulièrement qui présente les caractéristiques décrites ci-dessus) du sang S d'un individu à au moins un système de transfert 12 (plus particulièrement qui présente les caractéristiques décrites ci-dessus) du plasma P prélevé (plus particulièrement que contient le sang S prélevé et centrifugé) et, d'autre part, en une étape raccordement dudit au moins un système de transfert 12 à au moins un contenant de collecte 11 (plus particulièrement qui présente les caractéristiques décrites ci-dessus) et, d'autre part encore, en une étape de transfert du plasma P contenu dans ledit au moins un dispositif de prélèvement 1 vers ledit au moins un contenant de collecte 11, ceci au travers dudit au moins un système de transfert 12.

En ce qui concerne l'étape de transfert, celle-ci consiste à exercer une pression sur une partie du moyen d'entraînement 7 demeurant à l'intérieur du tube 3 de la seringue 2 dudit au moins un dispositif de prélèvement 1 (autre que la partie de ce moyen d'entraînement 7 qui avait été retirée hors dudit au moins un dispositif de prélèvement 1 après l'étape de prélèvement et avant l'étape de centrifugation).

Une telle pression permet de repousser le plasma P à partir dudit au moins un dispositif de prélèvement 1 vers ledit au moins un contenant de collecte 11, ceci au travers dudit au moins un système de transfert 12.

On observera que cette pression est exercée de manière manuelle (avec un doigt, notamment en début de transfert) et/ou avec la partie du moyen d'entraînement 7 préalablement retirée hors dudit au moins un dispositif de prélèvement 1, plus particulièrement après l'étape de prélèvement et avant l'étape de centrifugation.

On observera que, après l'étape de centrifugation et au cours de l'étape de collecte, ledit au moins un dispositif de prélèvement 1 est maintenu de manière verticale et en sorte que l'embout 4 de la seringue 2 dudit au moins un dispositif de prélèvement 1 soit orienté vers le haut.

De plus, la pression exercée sur la partie du moyen d'entraînement 7 demeurant à l'intérieur du tube 3 de la seringue 2 dudit au moins un dispositif de prélèvement 1 est exercée de manière verticale, vers le haut et/ou selon un mouvement ascendant.

## Revendications

1. Dispositif de prélèvement (1) du sang (S) d'un individu, ce dispositif de prélèvement (1) comporte au moins une seringue (2) comportant un tube (3), un embout (4) qui équipe le tube (3), et un piston (5) qui comporte, d'une part, un joint d'étanchéité (6) qui est mobile à l'intérieur du tube (3) et, d'autre part, un moyen d'entraînement (7), qui est mobile par rapport au tube (3), qui est solidaire en déplacement dudit joint d'étanchéité (6), et qui est configuré pour entraîner ledit joint d'étanchéité (6) en déplacement par rapport au tube (3), **caractérisé par le fait que** ledit moyen d'entraînement (7) comporte au moins une portion (9) qui est sécable.

2. Dispositif de prélèvement (1) selon la revendication 1, **caractérisé par le fait que** ladite au moins une portion sécable (9) est configurée pour être sécable sous l'effet d'un mouvement du moyen d'entraînement (7) autre qu'un mouvement d'entraînement du joint d'étanchéité (6) par ce moyen d'entraînement (7).

3. Dispositif de prélèvement (1) selon la revendication 1, **caractérisé par le fait que** le tube (3) et/ou le moyen d'entraînement (7) s'étend selon un axe d'extension (Xe ; Xe') tandis que ladite au moins une portion sécable (9) est configurée pour être sécable sous l'effet d'un effort exercé sur ledit moyen d'entraînement (7) selon une direction qui forme un angle non nul avec un tel axe d'extension (Xe ; Xe').

4. Dispositif de prélèvement (1) selon l'une quelconque des revendications précédentes, **caractérisé par le fait que** ladite au moins une portion sécable (9) est configurée pour être sécable manuellement.

5. Dispositif de prélèvement (1) selon l'une quelconque des revendications précédentes, **caractérisé par le fait que** ladite au moins une portion sécable (9) comporte au moins une zone de fragilité et/ou au moins une zone de rupture préférentielle et/ou au moins une zone d'amorce de rupture.

6. Dispositif de prélèvement (1) selon l'une quelconque des revendications précédentes, **caractérisé par le fait que** ladite au moins une portion sécable (9) est localisée à proximité du joint d'étanchéité (6).

7. Dispositif de prélèvement (1) selon l'une quelconque des revendications précédentes, **caractérisé par le fait que** le piston (5) comporte des moyens de solidarisation (8) qui sont configurés pour solidariser le joint d'étanchéité (6) et le moyen d'entraînement (7) tandis que le moyen d'entraînement (7) comporte une partie d'extrémité proximale (72) qui comporte une partie (80) des moyens de solidarisation (8) ainsi que ladite au moins une portion sécable (9).

8. Dispositif de prélèvement (1) selon l'une quelconque des revendications précédentes, **caractérisé par le fait que** le moyen d'entraînement (7) comporte une tige ou une lame dont au moins une partie comporte et/ou constitue ladite au moins une portion sécable (9).

9. Dispositif de collecte (10) du plasma (P) contenu dans le sang (S) d'un individu, ce dispositif de collecte (10) comporte, d'une part, au moins un dispositif de prélèvement (1) du sang (S) d'un individu, d'autre part, au moins un contenant de collecte (11) du plasma (P) contenu dans le sang (S) dudit individu et, d'autre part encore, au moins un système de transfert (12) qui est configuré pour transférer le plasma (P) du sang (S) contenu dans ledit au moins un dispositif de prélèvement (1) vers ledit au moins au moins un contenant de collecte (11), **caractérisé par le fait que** ledit au moins un dispositif de prélèvement (1) du sang (S) d'un individu est conforme à l'une quelconque des revendications précédentes.

10. Dispositif de collecte (10) selon la revendication 9, **caractérisé par le fait que** ledit au moins un système de transfert (12) comporte au moins, d'une part, un premier raccord (120) qui est configuré pour être raccordé audit au moins un dispositif de prélèvement (1) et, d'autre part, un deuxième raccord (121) qui est configuré pour être raccordé audit au moins un contenant de collecte (11) et, d'autre part encore, au moins un conduit de raccordement (122) qui raccorde le premier raccord (120) et le deuxième raccord (121).

11. Dispositif de collecte (10) selon l'une quelconque des revendications 9 ou 10, **caractérisé par le fait qu'**il se présente sous la forme d'un kit.

12. Installation de collecte du plasma (P) contenu dans le sang (S) d'un individu, **caractérisé par le fait qu'**elle comporte, d'une part, au moins un dispositif de collecte (10) conforme à l'une quelconque des revendications 9 à 11 et, d'autre part, une machine de centrifugation (13) du sang (S) prélevé par l'intermédiaire dudit au moins un dispositif de prélèvement (1) que comporte ledit au moins un dispositif de collecte (10).

13. Procédé de collecte du plasma (P) contenu dans le sang (S) d'un individu, ce procédé comporte :
- une étape de prélèvement du sang (S) d'un individu ;
- une étape de centrifugation du sang (S) prélevé ;
- une étape de collecte du plasma (P) contenu dans le sang (S) prélevé et centrifugé, **caractérisé par le fait que** :
- l'étape de prélèvement consiste à prélever du sang (S) à l'aide d'au moins un dispositif de prélèvement (1) conforme à l'une quelconque des revendications 1 à 8 ;
- après l'étape de prélèvement et avant l'étape de centrifugation, le procédé de collecte comporte une étape de division qui consiste à diviser ladite au moins une portion sécable (9) du moyen d'entraînement (7) du piston (5) de la seringue (2) dudit au moins un dispositif de prélèvement (1) et à retirer au moins une partie de ce moyen d'entraînement (7) hors dudit au moins un dispositif de prélèvement (1).

14. Procédé de collecte selon la revendication 13, **caractérisé par le fait que** l'étape de centrifugation du sang (S) consiste à centrifuger le sang (S) avec une accélération comprise entre 200 g et 700 g.

15. Procédé de collecte selon l'une quelconque des revendications 13 ou 14, **caractérisé par le fait que** l'étape de collecte du plasma (P) consiste, d'une part, en une étape de raccordement d'au moins un dispositif de prélèvement (1) du sang (S) d'un individu à au moins un système de transfert (12) du plasma (P) prélevé et, d'autre part, en une étape raccordement dudit au moins un système de transfert (12) à au moins un contenant de collecte (11) et, d'autre part encore, en une étape de transfert du plasma (P) contenu dans ledit au moins un dispositif de prélèvement (1) vers ledit au moins un contenant de collecte (11), ceci au travers dudit au moins un système de transfert (12), cette étape de transfert consistant à exercer une pression sur une partie du moyen d'entraînement (7) demeurant à l'intérieur du tube (3) de la seringue (2) dudit au moins un dispositif de prélèvement (1), une telle pression étant exercée de manière manuelle et/ou avec la partie du moyen d'entraînement (7) préalablement retirée hors dudit au moins un dispositif de prélèvement (1).
